# EUROPÄISCHE PATENTSCHRIFT

(11) **EP 1 039 875 B1**
(45) Veröffentlichungstag und Bekanntmachung des Hinweises auf die Patenterteilung: **30.10.2002**
(21) Anmeldenummer: 98966641.7
(22) Anmeldetag: 17.12.1998
(51) Int. Cl.: A61K 7/06

(54) **HAARBEHANDLUNGMITTEL ENTHALTEND BIOPOLYMERE, PANTHENOL UND MONO- UND/ODER DISACCHARIDE**
HAIR TREATMENT COMPOSITION CONTAINING BIOPOLYMERS, PANTHENOL AND MONO- AND/OR DISACCHARIDES
COMPOSITION POUR LE TRAITEMENT DES CHEVEUX CONTENANT DES BIOPOLYMERES, DU PANTHENOL ET DES MONO-ET/OU DISACCHARIDES

(30) Priorität: 22.12.1997 DE 19757214
(43) Veröffentlichungstag der Anmeldung: 04.10.2000
(73) Patentinhaber: Henkel Kommanditgesellschaft auf Aktien, 40589 Düsseldorf-Holthausen (DE)
(72) Erfinder: PRIEBE, Christian, D-42489 Wülfrath (DE); GODDINGER, Dieter, D-25336 Klein Nordende (DE); HOLLENBERG, Detlef, D-40699 Erkrath (DE)
(86) Internationale Anmeldenummer: EP9808313
(87) Internationale Veröffentlichungsnummer: WO99032071

(56) Entgegenhaltungen:
- EP-A- 0 749 749
- EP-A- 0 823 252
- WO-A-92/19218
- WO-A-99/24004
- DE-A- 2 627 419
- DE-A- 19 531 145
- US-A- 4 364 837
- DATABASE WPI Week 9248 Derwent Publications Ltd., London, GB; AN 92-395340 XP002111657 "Hair tonic cosmetic material having improved tonic effect - consists of acidic soln. contg. water-soluble natural saccharide(s), blood plasma and substitutes, and chitin and/or chitosan derivs." & JP 04 295412 A (JAPAN HAPPY), 20. Oktober 1992 (1992-10-20)

## Beschreibung

Die Erfindung betrifft ein Mittel enthaltend eine Wirkstoffkombination aus kationischen oder pseudokationischen Biopolymeren und Mono- und/ oder Disacchariden und Panthenol. Ein weiterer Gegenstand betrifft die Verwendung der Wirkstoffkombination zur Herstellung von Haarbehandlungsmitteln sowie gegen Spliß.

Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare in unerwünschter Weise beeinflussen können. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen.

Es wird daher seit langem empfohlen, die Haare in diesem Fall einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert. Derartige Mittel werden - zumeist mit relativ niedrigen Wirkstoffkonzentrationen - auch als im Haar verbleibende Präparate angeboten.

Weiterhin wurden in jüngster Zeit sogenannte Kombinationspräparate entwickelt, um den Aufwand der genannten mehrstufigen Verfahren, insbesondere bei der direkten Anwendung durch Verbraucher, zu verringern. Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können aber noch nicht alle Wünsche der Verbraucher erfüllen.

So ist beispielsweise die biologische Abbaubarkeit der quatemären Ammoniumverbindungen in einzelnen Fällen nicht voll befriedigend und ihre in vielen Fällen mangelnde Kompatibilität mit Aniontensiden schränkt die Formulierungsmöglichkeiten stark ein. Weiterhin können bei bestimmten Polymeren unerwünschte Kumulationen auf dem Haar auftreten.

Alkylphosphonate und Alkylphosphate sind zwar mit anionischen Tensiden kompatibel, verfügen aber nur über begrenzte pflegende Eigenschaften.

Überraschenderweise wurde nun gefunden, daß das erfindungsgemäße Mittel enthaltend kationische oder pseudokationische Biopolymere und Mono- und/ oder Disaccharide und Panthenol, sich hervorragend zur Haarpflege eignet. Die Wirkstoffkombination kann sowohl für sich als auch in Kombination mit bekannten Haarpflegemitteln eingesetzt werden. Insbesondere zur Verwendung gegen Spliß hat sich die erfindungsgemäße Kombination bewährt.

Gegenstand der vorliegenden Erfindung ist daher ein Mittel enthaltend
a) kationische oder pseudokationische Biopolymere und
b) Panthenol oder Panthenol-Ether, Panthenol-Ester, kationisch derivatisierte Panthenole und
c) ein Mono- und/oder Disaccharid.

Ein weiterer Gegenstand der vorliegenden Erfindung betrifft die Verwendung der Wirkstoffkombination in bekannten Haarbehandlungsmitteln sowie die Verwendung der Wirkstoffkombination zur Verminderung von Spliß.

Das erfindungsgemäße Mittel läßt sich mit den dem Fachmann geläufigen Methoden in bekannte Formulierungen einarbeiten und führt nach der Anwendung auf dem Haar zu einer deutlichen Verminderung des Splisses.

### Kationische oder pseudokationische Biopolymere

Besonders geeignete kationische Biopolymere, die im Sinne der Erfindung in Betracht kommen, stellen Hydrokolloide vom Typ der Chitosane dar. Chemisch betrachtet handelt es sich dabei um partiell deacetylierte Chitine unterschiedlichen Molekulargewichtes, die den - idealisierten - Monomerbaustein (I) enthalten:

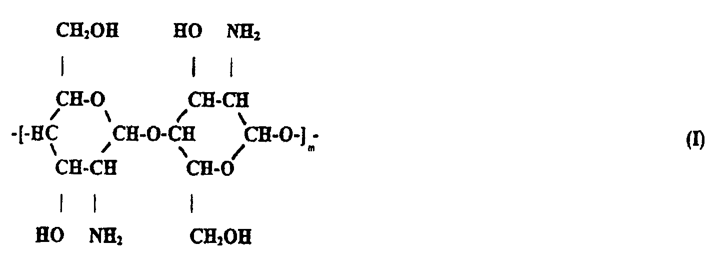

Im Gegensatz zu den meisten Hydrokolloiden, die im Bereich biologischer pH-Werte negativ geladen sind, stellen Chitosane unter diesen Bedingungen kationische Biopolymere dar.

Die positiv geladenen Chitosane können mit entgegengesetzt geladenen Oberflächen in Wechselwirkung treten, ihre Verwendung als Filmbildner in kosmetischen Haar- und Körperpflegemitteln ist daher in der Literatur bereits hinlänglich beschrieben (US 4,134,412; DE 2627419; EP 507 272).

Neben den Chitosanen können auch in einer weniger bevorzugten Ausrührungsform kationische Guar-Gumme im Rahmen der vorliegenden Erfindung eingesetzt werden. Es kann sich dabei beispielsweise um die unter den Handelsnamen Jaguar® und Cosmedia Guar® bekannten Verbindungen handeln.

Neben den Chitosanen kommen auch quaternierte, alkylierte und/oder hydroxyalkylierte Derivate, bevorzugt Succinate, gegebenenfalls auch in mikrokristalliner Form in Betracht. Der Einsatz erfolgt in der Regel in Form wäßriger Gele mit einem Feststoffgehalt im Bereich von 0,1 bis 5 Gew.-%.

Übersichten zu diesem Thema sind beispielsweise von B.Gesslein et al. in **HAPPI** 27, **57 (1990),** O.Skaugrud in **Drug Cosm. Ind.** 148, **24 (1991)** und E.Onsoyen et al. in **Seifen--Öle-Fette- Wachse** 117, **633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren zur Herstellung von - mikrokristallinem - Chitosan sind beispielsweise in der **WO 91/05808** (Firextra Oy) und der **EP-B1 0 382 150** (Hoechst) beschrieben.

In einer bevorzugten Ausführungsform der Erfindung werden besonders aschearme kationische Biopolymere eingesetzt, die man erhält, indem man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) das resultierende decalcifizierte dritte Zwischenprodukt schließlich mit konzentrierter wäßriger Alkalilauge behandelt und dabei bis zu einem Gehalt von 0,05 bis 0,5 und insbesondere 0, 15 bis 0,25 Mol Acetamid pro Mol Monomereinheit deacetyliert, und
(e) gegebenenfalls einer Druck/Temperaturnachbehandlung zur Einstellung der Viskosität unterwirft.

### Mono- und/ oder Disaccharide

Im Rahmen der vorliegenden Erfindung sind grundsätzlich alle Mono- und/ oder Disaccharide geeignet Vorzugsweise handelt es sich jedoch um Mono- und/ oder Disaccharide ausgewählt aus der Gruppe, die gebildet wird von D-Glucose, D-Galactose, D-Mannose, D-Fructose, L-Arabinose, D-Xylose, D-Ribose und D-Desoxyribose sowie Cellobiose, Maltose, Lactose und Saccharose. Insbesondere bevorzugt wird D-Glucose eingesetzt.

Das erfindungsgemäße Mittel weist üblicherweise einen sauren pH-Wert im Bereich von 2,5 bis 6 auf vorzugsweise einen leicht sauren pH-Wert, im Bereich von 4,5 bis 6. Zur Einstellung auf diesen pH-Wert lassen sich prinzipiell verschiedene Säuren, vorzugsweise Aminosäuren und/ oder Hydroxycarbonsäuren, insbesondere Milchsäure, Essigsäure und Citronensäure, einsetzten, vorzugsweise handelt es sich jedoch um Glutaminsäure. Besonders bevorzugt enthält das Mittel 0,1 bis 1, insbesondere 0,2 bis 0,6 Gew.-% der entsprechenden Säure.

Das erfindungsgemäße Mittel enthält vorzugsweise 0,1 bis 5, insbesondere 0,2 bis 1 Gew.-% kationischer oder pseudokationischer Biopolymere sowie vorzugsweise 0,1 bis 1, insbesondere 0,2 bis 0,5 Gew.-% an Mono- und/ oder Disacchariden. Das erfindungsgemäße Mittel umfaßt vorzugsweise eine wäßrige Lösung der Wirkstoffkombination, die jedoch bis zu 15 Gew.-% eines niederen Alkohols enthalten kann. Als niedere Alkohole im Sinne der vorliegenden Anmeldung kommen Alkohole mit 1 bis 4 C-Atomen in Frage, vorzugsweise Methanol, Ethanol und/ oder Isopropanol.

Das erfindungsgemäße Mittel enthält kationische oder pseudokationische Biopolymere kombiniert mit Mono- und/ oder Disacchariden und zusätzlich Panthenol. Es konnte bezüglich der Splißverminderung ein synergistischer Effekt durch zusätzlichen Einsatz von Panthenol festgestellt werden. Bevorzugt werden 0,1 bis 8, insbesondere 0,2 bis 5 Gew.-% Panthenol eingesetzt.

Unter Panthenol im Sinne der vorliegenden Anmeldung sind Verbindungen zu verstehen ausgewählt aus Panthenol, Panthenol-Ethern, Panthenol-Estern und kationisch derivatisierten Panthenolen.

Erfindungsgemäß verwendbare Panthenol-Ether bzw. Panthenolester sind beispielsweise das Triacetat des Panthenols sowie der Panthenolmonoethylether und dessen Monoacetat.

Bevorzugte Verbindungen sind aber das Panthenol selbst sowie kationische derivatisierte Panthenole.

Informationen bezüglich der Herstellung dieser Panthenol-Derivate sind der genannten Druckschrift WO 92/13829 zu entnehmen, auf die hier ausdrücklich Bezug genommen wird. Ein entsprechendes Produkt wird von der Firma Tri-K unter der Bezeichnung PANTHEQUAT^{R} vertrieben.

Das erfindungsgemäße Mittel läßt sich in alle üblichen Haarbehandlungsmittel problemlos einarbeiten. Die Haarbehandlungsmittel können alle für solche Mittel üblichen Komponenten enthalten, wobei die Auswahl dieser Komponenten im wesentlichen durch die Art des Haarbehandlungsmittels bestimmt wird.

In einer bevorzugten Ausführungsform enthalten die Haarbehandlungsmittel **Fettalkohole.** Unter Fettalkoholen sind primäre aliphatische Alkohole der Formel (**II**) zu verstehen,

**R**^{**1**}**OH (II)**

in der R¹ für einen aliphatischen, linearen oder verzweigten Kohlenwasserstoffrest mit 6 bis 22 Kohlenstoffatomen, der gesättigt ist oder bis zu 3 Doppelbindungen enthalten kann, steht.

Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Bevorzugt sind technische Fettalkohole mit 12 bis 18 Kohlenstoffatomen wie beispielsweise Kokos-, Palm-, Palmkern- oder Talgfettalkohol. Vorzugsweise enthalten die Mittel 0,5 bis 50, insbesondere 2 bis 20 Gew.-% Fettalkohol.

Neben den Fettalkoholen können vorzugsweise auch noch Tenside in den erfindungsgemäßen Haarbehandlungsmitteln enthalten sein. Es handelt sich dabei in einer bevorzugten Ausführungsform um kationische Tenside, Seifen und/ oder Dicarbonsäuren.

Als **kationische Tenside** kommen beispielsweise quartäre Ammoniumverbindungen, Amidoamine sowie quaternisierte Ester und Proteinhydrolysate in Frage.

**Dicarbonsäuren** im Sinne der vorliegenden Anmeldung sind beispielsweise Verbindungen der Formel **(III),** 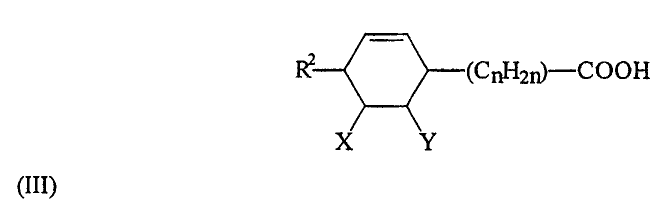 in der R² steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (III), die zusätzlich noch 1 bis 3 Methyl- oder Ethylsubstituenten am Cyclohexenring tragen, Dicarbonsäuren, die aus den Dicarbonsäuren gemäß Formel (III) formal durch Anlagerung eines Moleküls Wasser an die Doppelbindung im Cyclohexenring entstehen, oder einem physiologisch verträglichen Salz einer dieser Säuren. Ein Herstellungsverfahren für diese Dicarbonsäuren ist beispielsweise der US-Patentschrift 3,753,968 zu entnehmen.

Es sind solche Dicarbonsäuren der Formel (III) bevorzugt, bei denen R² steht für eine lineare oder methylverzweigte, gesättigte Alkylgruppe mit 4 bis 8 Kohlenstoffatomen und n für eine Zahl von 6 bis 10.

Erfindungsgemäß einsetzbar neben den, bevorzugten, Dicarbonsäuren gemäß Formel (III) sind auch solche Dicarbonsäuren, die sich von den Verbindungen gemäß Formel (III) durch 1 bis 3 Methyl- oder Ethyl-Substituenten am Cyclohexylring unterscheiden oder aus diesen Verbindungen formal durch Anlagerung von einem Molekül Wasser an die Doppelbildung des Cyclohexenrings gebildet werden.

Als erfindungsgemäß besonders wirksam hat sich die Dicarbonsäure(-mischung) erwiesen, die durch Umsetzung von Linolsäure mit Acrylsäure entsteht. Es handelt sich dabei um eine Mischung aus 5- und 6-Carboxy-4-hexyl-2-cyclohexen-1-octansäure, Solche Verbindungen sind kommerziell unter den Bezeichnungen Westvaco Diacid® 1550 und Westvaco Diacid® 1595 (Hersteller: Westvaco) erhältlich.

Neben den Dicarbonsäuren selbst können auch deren physiologisch verträgliche Salze erfindungsgemäß eingesetzt werden. Beispiele für solche Salze sind die Alkali-, Erdalkali-, Ammonium-, die Mono-, Di- und Trimethyl-, -ethyl- und -hydroxyethyl-Ammoniumsalze sowie das Zinksalz. Die Natrium-, Kalium- und Ammoniumsalze sind bevorzugte Salze. Weiterhin kann es aus Formulierungsgründen bevorzugt sein, die Dicarbonsäure aus den wasserlöslichen Vertretern, insbesondere den wasserlöslichen Salzen auszuwählen. Das Kaliumsalz der 5- und 6-Carboxy-4-hexyl-2-cyclohexen-1-octansäure, das als wäßrige Lösung unter der Bezeichnung Westvaco Diacid® H-240 kommerziell erhältlich ist, hat sich als besonders geeignet erwiesen.

Weiterhin bevorzugt werden zusätzlich **wasserlösliche Polymere** eingesetzt. Dabei kann es sich im Sinne der vorliegenden Erfindung sowohl um nichtionische, kationische und/oder amphotere Polymere handeln. Bevorzugte Polymere sind die nichtionogenen Polymere wie beispielsweise Polyvinylpyrrolidon, Vinylpyrrolidon/Vinylacetat-Copolymere und Celluloseether, Stärke und/ oder Cyclodextrine besonders bevorzugt sind Polymere die unter den Handelsnamen Luviquat®, Merquat® und Mirapol® erhältlich sind.

Eine weitere Klasse fakultativer Komponenten stellen die **nichtionogenen Tenside** dar.

Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bei diesen Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den nichtionogenen Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Alkylpolyglykoside gemäß der Formel RO-(Z)ₓ, in der R steht für einen Alkylrest mit 8 bis 22 Kohlenstoffatomen, Z für ein Mono- oder Oligosaccharid und x für eine Zahl von 1,1 bis 5, sind besonders bevorzugte nichtionogene Tenside. Diese Verbindungen sind durch die folgenden Parameter gekennzeichnet.

Der Alkylrest R enthält 8 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂-bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylglykoside mit x-Werten von 1,3 bis 2 sind bevorzugt. Ganz besonders bevorzugt sind Alkylglykoside, bei denen x 1,4 bis 1,6 beträgt.

Unter Haarbehandlungsmittel fallen auch jene Mittel zum Färben und Tönen der Haare, solche Mittel enthalten entweder sogenannte "direktziehende" Farbstoffe und/oder Vorprodukte für Oxidationsfarbstoffe.

Übliche **direktziehende Farbstoffe** sind z. B. Nitrobenzolderivate, Antrachinon-Farbstoffe, Triphenylmethanfarbstoffe und Azofarbstoffe. In Haarfärbe- und Tönungsmitteln eingesetzte Vertreter dieser Farbstoffklasse sind beispielsweise die unter den internationalen Bezeichnungen bzw. Handelsnamen HC Yellow 2, HC Yellow 4, Methylgelb, Fluorgelb, Basic Yellow 57, Disperse Orange 3, HC Red 3, HC Red BN, Kardinalrot, Rot Y, Rot X, Basic Red 76, HC Blue 2, Nitroblau, Disperse Blue 3, Basis Blue 99, HC Violet 1, Disperse Violet 1, Disperse Violet 4, Disperse Black 9, Basis Brown 16, Pikraminsäure und Rodol 9 R bekannten Verbindungen. Weitere geeignete direktziehende Farbstoffe sind beispielsweise die Verbindungen 3-Nitro-4-ethylaminobenzoesäure, 1,2,3,4-Tetrahydro-6-nitro-chinoxalin, 2-Nitro-4-amino-4'dimethyl-aminodiphenylamin-2'-carbonsäure und 2-Nitro-4-aminodiphenylamin-2'carbonsäure.

In einer bevorzugte Ausführungsform können auch Indole und/ oder Indoline enthalten sein, im Sinne der vorliegenden Erfindung besonders bevorzugt sind 6-Hydroxyindol, N-Methyl-6-hydroxyindol, N-Ethyl-6-hydroxyindol, N-Propyl-6-hydroxyindol, N-Butyl-6-hydroxyindol, 4-Hydroxyindol, N-Methyl-4-hydroxyindol, N-Ethyl-4-hydroxyindol, N-Propyl-4-hydroxyindol, N-Butyl-4-hydroxyindol, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol sowie 5,6-Dihydroxyindolin, N-Methyl-5,6-dihydroxyindolin, N-Ethyl-5,6-dihydroxyindolin, N-Propyl-5,6-dihydroxyindolin und N-Butyl-5,6-dihydroxyindolin.

Weiterhin können die erfindungsgemäßen Zubereitungen auch in der Natur vorkommende Farbstoffe wie beispielsweise Henna rot, Henna neutral, Henna schwarz. Kamillenblüte. Sandelholz, schwarzen Tee, Faulbaumrinde, Salbei, Blauholz, Krappwurzel, Catechu, Sedre und Alkannawurzel enthalten.

Als Vorprodukte für Oxidationsfarbstoffe enthalten Haarfarbemittel sogenannte Entwickler- und Kupplerkomponenten. Die Entwicklerkomponenten bilden unter dem Einfluß von Oxidationsmitteln oder Luftsauerstoff untereinander oder unter Kupplung mit einer oder mehreren Kupplerkomponenten die eigentlichen Farbstoffe aus.

Als **Entwicklerkomponenten** werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Spezielle Vertreter sind beispielsweise p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol.

Als **Kupplerkomponenten** werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl3-methyl-pyrazolon-5, 2,4-Dichlor-3-aminophenol, 1,3-Bis-(2,4-diaminophenoxy)-propan, 2-Chlor-resorcin, 2-Chlor-6-methyl-3-aminophenol und 2-Methylresorcin.

Die Art der erfindungsgemäßen kosmetischen Mittel unterliegt keinen Beschränkungen. Die erfindungsgemäßen Mittel können sowohl auf dem Haar verbleiben (leave-on), als auch nach einer Einwirkzeit von wenigen Sekunden bis Minuten wieder ausgespült werden (rinse-off). Bevorzugt ist der Einsatz der erfindungsgemäßen Wirkstoffkombination in den sogenannten *rinse-off* Produkten. Beispiele für erfindungsgemäße Mittel sind Shampoos, Spülungen, Kuren, Konditioniermittel. Tönungsmittel, Färbemittel, Dauerwellmittel, Fixiermittel. Haarsprays und Fönwellen.

Weitere Komponenten dieser Mittel können dann beispielsweise sein:
- zwitterionische Tenside, wie beispielsweise Betaine,
- Alkylethercarbonsäuren,
- ampholytische Tenside,
- Strukturanten wie Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle und Dimethylisosorbid,
- Lösungsvermittler, wie Ethanol, Isopropanol, Ethylenglykol, Propylenglykol, Glycerin und Diethylenglykol,
- Farbstoffe,
- weitere Substanzen zur Einstellung des pH-Wertes,
- Wirkstoffe wie Allantoin, Pyrrolidoncarbonsäuren und deren Salze, Pflanzenextrakte und Vitamine,
- Lichtschutzmittel,
- Konsistenzgeber wie Zuckerester, Polyolester oder Polyolalkylether,
- Wachse, wie Bienenwachs und Montanwachs,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex und Polyvinylacetat,
- Perlglanzmittel wie Ethylenglykolmono- und -distearat,
- Treibmittel wie Propan-Butan-Gemische, N₂O, Dimethylether, CO₂ und Luft sowie
- Antioxidantien,
- Reduktionsmittel wie z. B. Thioglykolsäure und deren Derivate, Thiomilchsäure, Cysteamin, Thioäpfelsäure und α-Mercaptoethansulfonsäure.

Die erfindungsgemäßen Mittel können beispielsweise als wäßrige, alkoholische oder wäßrig-alkoholische Lösungen, Cremes, Gele oder Emulsionen formuliert sein.

Es hat sich gezeigt, daß praktisch unabhängig von der Art des Haarbehandlungsmittels bestimmte weitere Komponenten die erfindungsgemäße Wirkstoffkombination in ihrer Wirkung besonders vorteilhaft ergänzen.

Als besonders vorteilhaft haben sich Haarbehandlungsmittel erwiesen, die neben der erfindungsgemäßen Wirkstoffkombination Acyllactylate der allgemeinen Formel (IV) 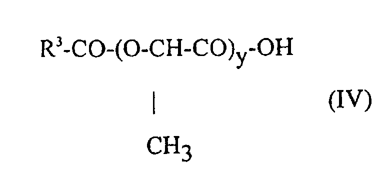 in der R³ für eine lineare oder verzweigte, gesättigte oder ungesättigte Alkyl- oder Alkenylgruppe mit 6 bis 22 Kohlenstoffatomen steht und y für eine Zahl von 1 bis 5 enthalten.

Acyllactylate, bei denen R³ für eine, insbesondere gesättigte, lineare oder methylverzweigte Alkylgruppe mit 12 bis 18 Kohlenstoffatomen steht und y für eine Zahl von 1 bis 3, sind besonders bevorzugt.

Solche Acyllactylate sind im Handel unter dem Warenzeichen Pationic® erhältlich.

Ein Natrium-isostearoyllactyllactylat, das unter der Bezeichnung Pationic®ISL vertrieben wird, ist ein besonders bevorzugtes Acyllactylat.

Weiterhin bevorzugt in erfindungsgemäß verwendeten haarkosmetischen Mitteln einzusetzende Substanzen sind beispielsweise:
- Antischuppenwirkstoffe wie Climbazol, Piroctone Olamine und Zink Omadine,
- haarkonditionierende Verbindungen wie Phospholipide, beispielsweise Sojalecithin, Ei-Lecitin und Kephaline, sowie Silikonöle,
- Oxidationsmittel wie Wasserstoffperoxid, Kaliumbromat und Natriumbromat.

## Patentansprüche

1. Mittel enthaltend
a) kationische oder pseudokationische Biopolymere und
b) Panthenol oder Panthenol-Ether, Panthenol-Ester, kationisch derivatisierte Panthenole und
c) ein Mono- und/oder Disaccharid.

2. Mittel nach Anspruch 1, **dadurch gekennzeichnet, daß** es sich um eine wäßrige Lösung mit einem pH-Wert im Bereich von 2,5 bis 6 handelt.

3. Mittel nach Anspruch 2, **dadurch gekennzeichnet, daß** der pH-Wert mit einer Aminound/oder Hydroxycarbonsäure eingestellt wird.

4. Mittel nach einem der Ansprüche 1 bis 3, **dadurch gekennzeichnet, daß** es 0,1 bis 5 Gew.-% kationische oder pseudokationische Biopolymere und 0,1 bis 8 Gew.-% Panthenol enthält.

5. Mittel nach einem der Ansprüche 1 bis 4, **dadurch gekennzeichnet, daß** es 0,1 bis 1 Gew.-% Mono- und/oder Disaccharide enthält.

6. Verwendung einer Wirkstoffkombination nach einem der Ansprüche 1 bis 5 zur Herstellung von Haarbehandlungsmitteln.

7. Verwendung der Wirkstoffkombination nach einem der Ansprüche 1 bis 5 zur Splißverminderung.

## Claims

1. A composition containing
a) cationic or pseudocationic biopolymers and
b) panthenol or panthenol ethers, panthenol esters, cationically derivatized panthenols and
c) a mono- and/or disaccharide.

2. A composition as claimed in claim 1, **characterized in that** it is an aqueous solution with a pH value of 2.5 to 6.

3. A composition as claimed in claim 2, **characterized in that** the pH is adjusted with an amino- and/or hydroxycarboxylic acid.

4. A composition as claimed in any of claims 1 to 3, **characterized in that** it contains 0.1 to 5% by weight of cationic or pseudocationic biopolymers and 0.1 to 8% by weight of panthenol.

5. A composition as claimed in any of claims 1 to 4, **characterized in that** it contains 0.1 to 1% by weight of mono- and/or disaccharides.

6. The use of the active-substance combination claimed in any of claims 1 to 5 for the production of hair treatment formulations.

7. The use of the active-substance formulation claimed in any of claims 1 to 5 for reducing split ends.

## Revendications

1. Composition contenant
a) des biopolymères cationiques ou pseudocationiques et
b) du panthénol ou de l'éther de panthénol, de l'ester de panthénol, des panthénols à dérivation cationique et
c) un mono- et/ou disaccharide.

2. Composition selon la revendication 1, **caractérisée en ce qu'**il s'agit d'une solution aqueuse présentant un pH compris dans l'intervalle de 2,5 à 6.

3. Composition selon la revendication 2, **caractérisée en ce que** le pH est ajusté au moyen d'un acide aminé et/ou d'un acide hydroxycarboxyli-que.

4. Composition selon une des revendications 1 à 3, **caractérisée en ce qu'**elle contient 0,1 à 5 % en poids de biopolymères cationiques ou pseudocationiques et 0,1 à 8 % en poids de panthénol.

5. Composition selon une des revendications 1 à 4, **caractérisée en ce qu'**elle contient 0,1 à 1 % en poids de mono- et/ou disaccharides.

6. Utilisation d'une association de substances actives selon une des revendications 1 à 5 pour fabriquer des produits de traitement capillaire.

7. Utilisation de l'association de substances actives selon une des revendications 1 à 5 pour diminuer le fourchage.
